# EUROPÄISCHE PATENTANMELDUNG

(11) **EP 1 129 677 A2**
(43) Veröffentlichungstag der Anmeldung: **05.09.2001**
(21) Anmeldenummer: 01103714.0
(22) Anmeldetag: 15.02.2001
(51) Int. Cl.: A61F 2/46

(54) **Instrumentarium zur Herstellung der Befestigungsflächen für eine Kniegelenkendoprothese**

(30) Priorität: 29.02.2000 DE 10009697
(71) Anmelder: Brehm, Peter, 91085 Weisendorf (DE)
(72) Erfinder: Müller, Peter, 91462 Dachsbach (DE)
(74) Vertreter: Schneck, Herbert, Dipl.-Phys., Dr.

(57) **Zusammenfassung**

Bei einem Instrumentarium zur Herstellung der Befestigungsflächen zur Befestigung einer Kniegelenkendoprothese an dem knienahen Oberschenkel und am Tibiakopf, ist vorgesehen, einen rotierenden Fräskopf (2) relativ zur Längsachse von Femur (40) bzw. Tibia mittels Befestigungseinrichtungen (Knochenklammer 26) festlegbar und relativ zu dieser Längsachse unter Definition von Fräsebenen (Schnittebenen I bis V) durch Verschwenken endlagenstabil winkeleinstellbar und in der jeweiligen so eingestellten Fräsebene (Schnittebenen I bis V) diese abdeckend als Stirn- bzw. Walzenfräser arbeitend verfahrbar zu lagern.

## Beschreibung

Die Erfindung richtet sich auf ein Instrumentarium zur Herstellung der Befestigungsflächen für eine Kniegelenkendoprothese an dem knienahen Oberschenkel und am Tibiakopf.

Eine Kniegelenkprothese der in Betracht stehenden Art wird beispielsweise beschrieben in DE 41 02 509 C2. In der dortigen Fig. 3 sind insbesondere auch die Befestigungsflächen erkennbar, die zur Implantation des femur-seitigen Prothesenteils, also zur Festlegung am knieseitigen Ende des Oberschenkels, erforderlich sind. Diese Mehrzahl von im Winkel zueinander angeordneten Befestigungsflächen wird herkömmlicherweise dadurch hergestellt, daß nach Freilegung der Knochen eine in axialer Richtung des Knochens verlaufende Markraumstange eingesetzt wird und mit Hilfe einer hieran und direkt am Knochen befestigten Sägeschablone mittels einer Säge nacheinander die einzelnen Ebenen gesägt werden.

Um die Schablonen beim Sägevorgang unverrutschbar zu positionieren, ist ein sehr hoher Aufwand erforderlich und gleichwohl gelingt es letztendlich nicht, eine wirklich perfekte und unverrutschbare Positionierung zu erzielen. Darüber hinaus sind die erforderlichen Arbeitsgänge aufwendig und führen zu einer verhältnismäßig hohen Ungenauigkeit und unnötig langer Operationsdauer, die den Patienten ebenso wie letztendlich auch den Operateur belastet. Die herkömmliche Erstellung dieser Befestigungsflächen wird beispielsweise ausführlich beschrieben in der Firmenschrift "MC-KNIEGELENK nach Prof. Dr. Diehl", dort Seite 5 ff.

Hiervon ausgehend liegt der Erfindung die Aufgabe zugrunde, ein Instrumentarium zu schaffen, welches eine einfachere, schnellere und genauere Realisierung der für die Implantation des Tibiateils und insbesondere des Femurteils einer Prothese erforderlichen Befestigungsflächen ermöglicht.

Diese Aufgabe wird erfindungsgemäß gelöst durch einen rotierenden Fräskopf, der relativ zur Längsachse von Femur bzw. Tibia mittels Befestigungseinrichtungen festlegbar und relativ zu dieser Längsachse unter Definition von Fräsebenen durch Verschwenken endlagenstabil winkeleinstellbar und in der gestellten Fräsebene diese abdeckend als Stirn- bzw. Walzenfräser arbeitend verfahrbar gelagert ist.

Durch die erfindungsgemäße Ausgestaltung entfallen die grundsätzlichen Probleme, die sich beim herkömmlichen Sägen unter Zuhilfenahme einer Schablone ergeben. Darüber hinaus ist eine Nach- und Feinbearbeitung (Nachjustierung) möglich, wenn dies erforderlich oder wünschenswert erscheint.

Vorteilhafterweise umfassen die Befestigungseinrichtungen den jeweiligen Knochen umgreifende Klammern.

Dabei kann eine erste Klammer den Knochen von oben übergreifen, wobei die Klammer zwei Backen aufweist, die relativ zueinander verschiebbar und endlagenfixierbar sind.

Die Befestigungseinrichtungen können auch noch wenigstens eine zweite Klammer umfassen, deren Schenkel annähernd parallel zur Knochenlängsachse verlaufen und relativ zueinander, verfahrbar sind, wobei an den Schenkelenden Befestigungsdorne und/oder -bohrungen zum Einbringen von Befestigungsschrauben ausgebildet sind.

Die demnach vorgesehenen Dorne ermöglichen eine Vorjustierung zum Bohren von Löchern für die Befestigungsschrauben im Knochen bzw. während des Einschraubvorganges. Die Fräsvorrichtung kann jederzeit von dem Fixierungsanteil gelöst werden und zu einem späteren Zeitpunkt wieder exakt in ihrer Ausgangslage durch Schnellverschlüsse befestigt werden.

Vorzugsweise ist der Fräskopf höhenverstellbar ausgebildet, so daß zur Ausbildung einer bestimmten Befestigungsfläche eine entsprechende Einstellmöglichkeit besteht. Die Höhenverstellung kann günstigerweise durch eine endlagenstabile oder stabilisierbare Gewindespindel realisiert werden.

Bei einer bevorzugten Ausführungsform ist vorgesehen, daß der Fräskopf bei eingestelltem Fräswinkel über eine Schienenführung linear translatorisch verlagerbar und über ein Schwenklager oder Kreuzführung führbar ist, so daß hierdurch eine Fräsebene definiert wird, die von dem Fräskopf nacheinander abgearbeitet werden kann. Der Fräskopf hat dabei eine zylinderförmige Konfiguration, wobei die Zylinderoberfläche die Fräsfläche bildet und wobei die Stirnseite des Zylinders vorzugsweise parallel zur Fräsebene verläuft.

Vorteilhafterweise ist weiterhin eine Voreinstelleinrichtung für die Beinachsenstellung vorgesehen, so daß auf diese Weise berücksichtigt werden kann, ob der Patient Varus-Valgus-Fehlstellung aufweist. Ebenso kann die Beinrotation genau (Varus oder Valgus-Fehlstellung) korrigiert und eingestellt werden.

Darüber hinaus sind Einstelleinrichtungen für die Beinlänge und für die Prothesengröße vorgesehen.

Im Rahmen der Erfindung ist es möglich, den Fräskopf bzw. den Fräser manuell unter Benutzung der erfindungsgemäßen Führung zu bewegen.

Alternativ kann aber insbesondere auch ein servomotorischer Antrieb für die Einstelleinrichtungen und/oder die Bewegung des Fräskopfes in der Fräsebene vorgesehen sein, wobei dann günstigerweise die Ansteuerung der Servomotoren computergesteuert erfolgt. Es ist dementsprechend möglich, die ganze Operation computergesteuert vorzunehmen, insbesondere auch in Abhängigkeit von vorher hergestellten, in den Computer abgespeicherten Röntgenbildern oder CT-Bildern.

Zur Navigation können an dem Instrumentarium Infrarotmarker vorgesehen sein, um alternativ zu einer manuellen Handhabung und Einstellung des Fräskopfes eine computergesteuerte Einstellung in Verbindung mit einer dreidimensionalen Computeranimation am Bildschirm, gewonnen aus einer vorher durchgeführten Computertomographie, vorzunehmen.

Nachfolgend wird die Erfindung anhand eines bevorzugten Ausführungsbeispiels in Verbindung mit der Zeichnung näher erläutert. Dabei zeigen:
- Fig. 1: eine Ansicht von oben des erfindungsgemäßen Instrumentariums, wobei der Fräser horizontal steht,
- Fig. 2: eine Fig. 1 entsprechende Darstellung, wobei der Fräser vertikal steht,
- Fig. 3: eine Ansicht von der Seite, wobei der Fräser lotrecht auf der Schnittebene I steht,
- Fig. 4: eine Ansicht von der Seite entsprechend Fig. 3, wobei der Fräser lotrecht auf der Schnittebene II steht,
- Fig. 5: eine entsprechende Ansicht von der Seite, wobei der Fräser lotrecht auf der Schnittebene III steht,
- Fig. 6: eine entsprechende Ansicht von der Seite, wobei der Fräser parallel zur Schnittebene IV steht,
- Fig. 7: eine Ansicht von der Seite, wobei der Fräser parallel zur Schnittebene V steht,
- Fig. 8: eine Ansicht von vorne, wobei der Fräser vertikal steht, und
- Fig. 9a bis 9f: einen Femurknochen und die Relativlage des Fräsers zu diesem Femurknochen sowie die an dem Femurknochen mittels des Fräsers hergestellten Schnittebenen I bis V.

In der Zeichnung ist ein Fräser 1 dargestellt, der einen drehantreibbaren Fräskopf 2 aufweist, welcher sowohl über seine Mantelfläche 3 als Walzenfräser als auch über seine Stirnfläche 4 als Stirnfräser einsetzbar ist. Bei der nachfolgend beschriebenen Erstellung der Schnittebenen I bis III wird der Fräskopf 2 als Stirnfräser und bei der Erstellung der Schnittebenen IV und V als Walzenfräser eingesetzt.

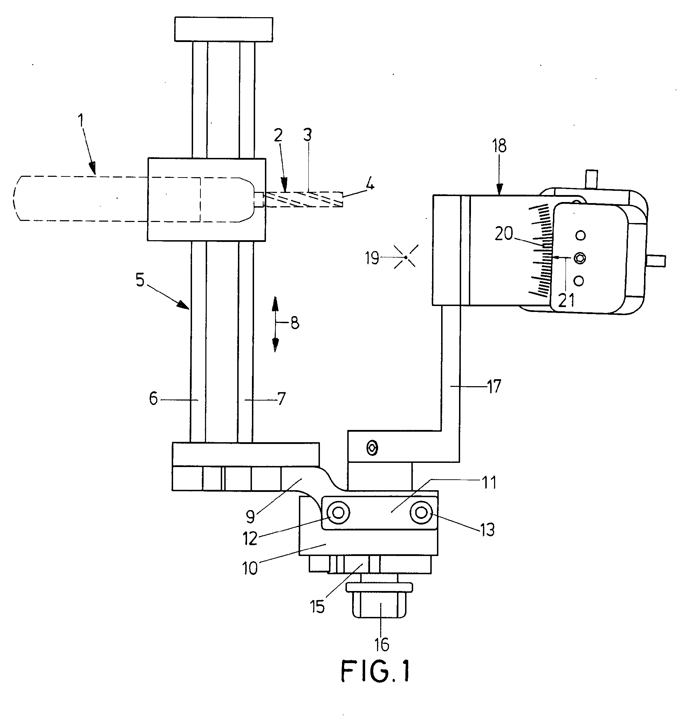

Der Fräser 1 ist auf einer x-Führung 5 umfassend zwei zueinander parallele Schienen 6, 7 in Richtung des Doppelpfeils 8 in Fig. 1 längs verfahrbar.

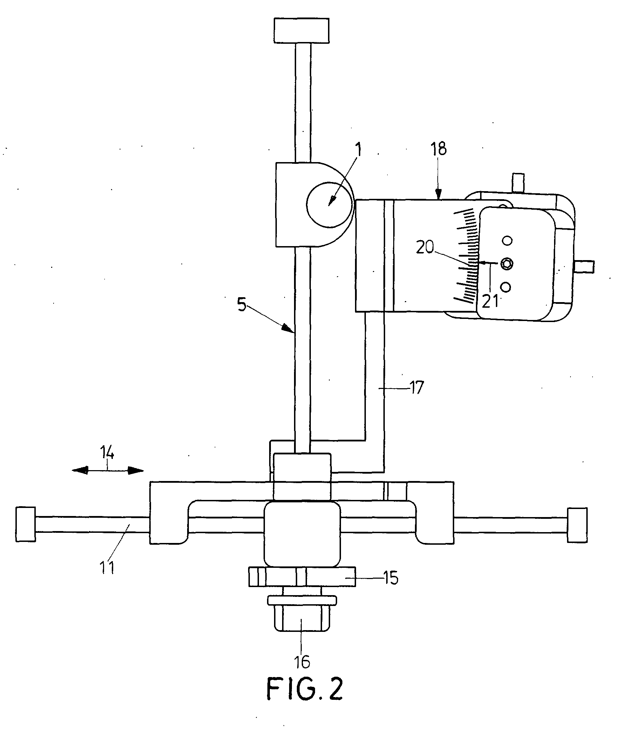

Die X-Führung 5 ist über eine Halterung 9 an einem Lagerbock 10 befestigt, von welchem sich eine y-Führung 11 senkrecht zur X-Führung 5 umfassend ebenfalls zwei Schienen 12, 13 weg erstreckt, die ein Verfahren des Fräsers 1 in Richtung des Doppelpfeils 14 in Fig. 2 ermöglicht, also eine Hoch-Tief-Verstellung.

Am Lagerbock 10 ist weiterhin eine Steuerscheibe 15 vorgesehen, welche verschiedene endlagenstabile Schwenkwinkeleinstellungen entsprechend den Fräsebenen I bis V ermöglicht, die durch eine Klemmschraube 16 fixierbar sind.

Weiterhin ist mit dem Lagerbock 10 über einen Arm 17 ein Einstellblock 18 verbunden; der ein Verschwenken um eine außerhalb des Einstellblocks 18 liegende Schwenkachse 19 ermöglicht, wobei eine Skala 20 in Verbindung mit einer feststehenden Markierung 21 eine Korrektureinstellung zur Berücksichtigung einer X- bzw. O-Beinstellung (Varus-Valgus-Korrektur) ermöglicht. Der Vorteil dieser Korrekturanordnung liegt darin, daß bei Vornahme einer Einstellung bzw. Verstellung die anderen Achsen nicht beeinträchtigt werden.

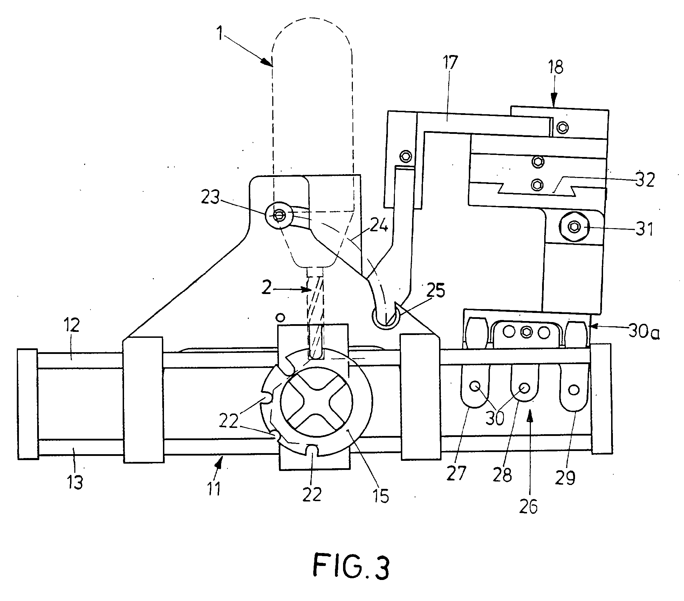

In Fig. 3 sind in der Steuerscheibe 15 Ausnehmungen 22 zur Einstellung der Fräsebenen I bis V erkennbar. Weiterhin ist dort eine Fräserarretierung 23 dargestellt, um den Fräser 1 als Stirnfräser zu verwenden, wobei er längs der Kurvenbahn 24 um 90° in die Fräserarretierung 25 verschwenkbar ist, um dort als Walzenfräser zu arbeiten.

Weiterhin erkennbar ist in Fig. 3 eine Knochenklammer 26 umfassend drei Klammeransätze 27, 28, 29 mit jeweils vorspringenden Dornen 30, wobei diese Knochenklammer 26 über einen Schnellverschluß 30a leicht abnehmbar ist.

Weiterhin gut erkennbar ist in Fig. 3 eine Stellschraube 31 für die Verstellung anterior-posterior sowie eine Schwalbenschwanzführung 32 für die Verstellung medial-lateral.

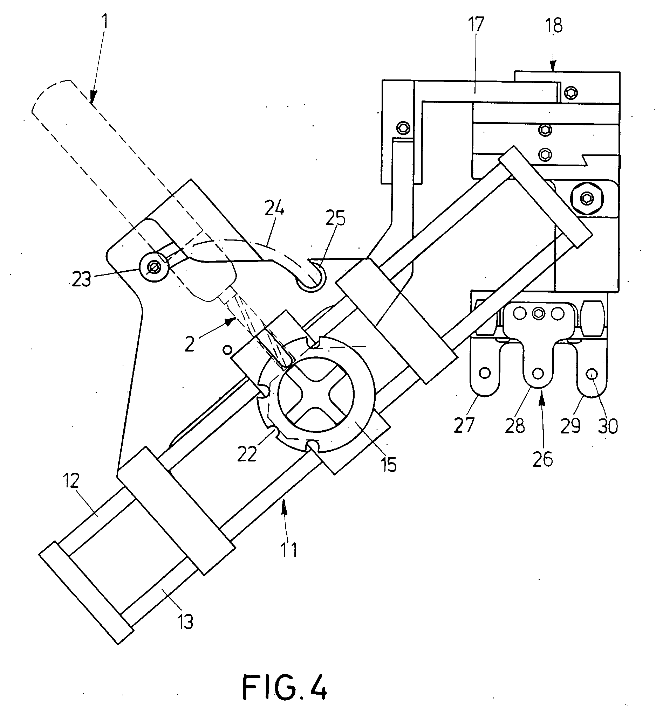

Fig. 4 zeigt eine Fig. 3 entsprechende Ansicht, wobei der Fräser 1 nun lotrecht auf der Schnittebene II steht und diese im Wege des Stirnfräsens erstellt.

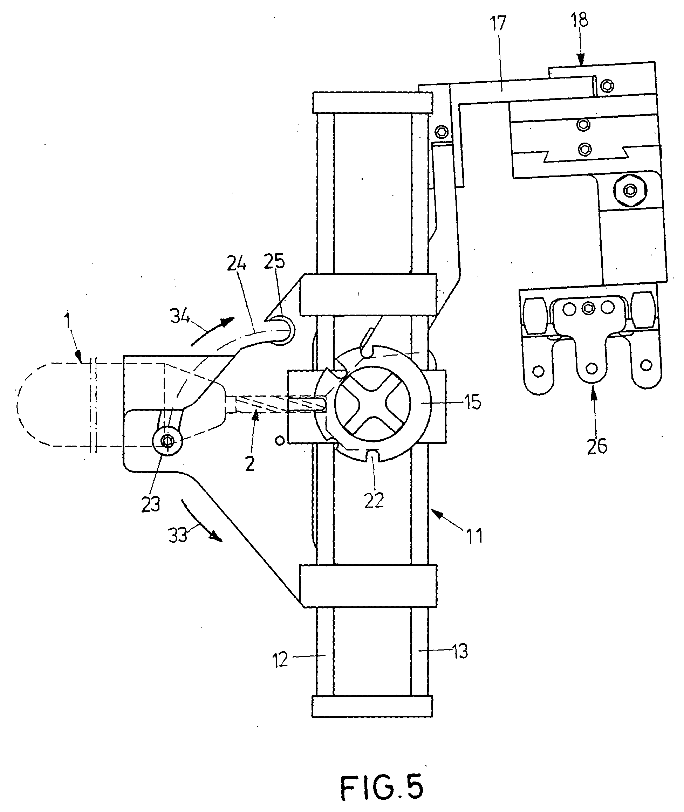

Bei der in Fig. 5 als Seitenansicht dargestellten Position erfolgt die Erstellung der Schnittebene III im Wege des Stirnfräsens, d.h. der Fräser 1 steht lotrecht auf der Schnittebene III.

Anhand des Pfeils 33 ist veranschaulicht, daß der als Femurfräser arbeitende Fräser 1 durch ein entsprechendes Verschwenken in Richtung dieses Pfeils 33 auch als Tibia-Fräser eingesetzt werden kann.

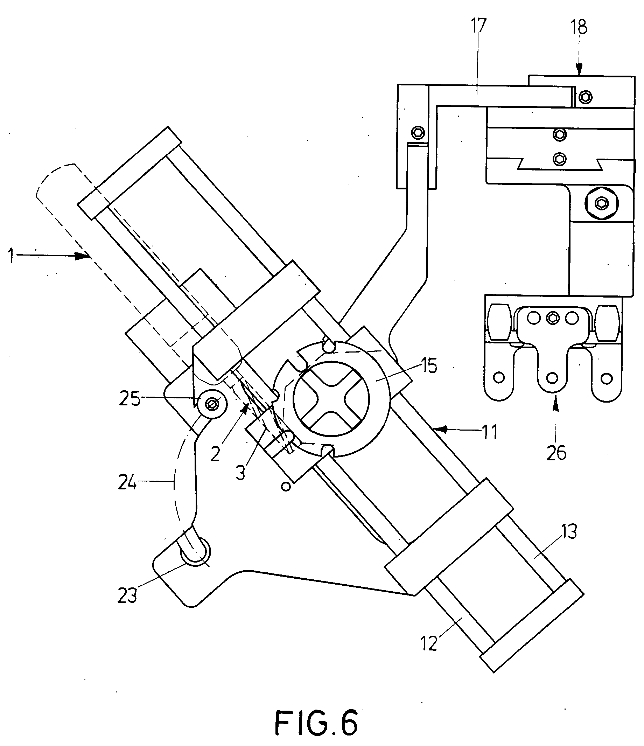

Ausgehend von der in Fig. 5 dargestellten Position erfolgt ein Verschwenken des Fräsers 1 bzw. des Fräskopfes 2 längs der Bahn 24 in Richtung des Pfeils 34 in die in Fig. 6 dargestellte Position, wo der Fräser 1 nun unter Einsatz seiner Mantelfläche 3 als Walzenfräser zur Erstellung der Schnittebene V eingesetzt wird, indem er nun parallel zu dieser Schnittebene V geführt wird.

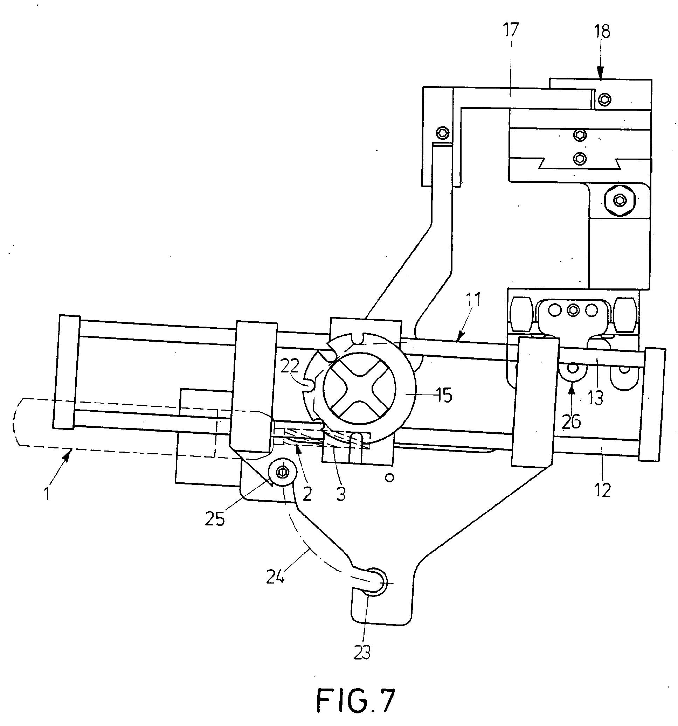

Bei der in Fig. 7 dargestellten Einstellung arbeitet der Fräser 1 ebenfalls als Walzenfräser, indem er parallel zur Schnittebene diese herstellend verfahren wird.

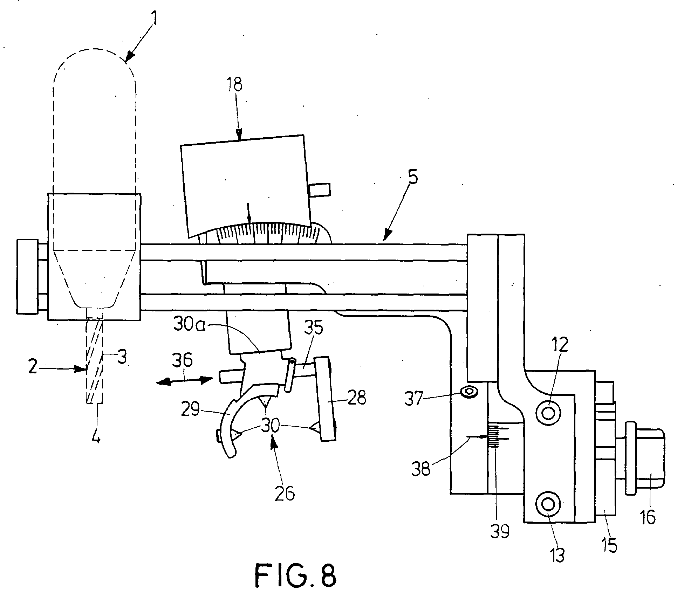

Fig. 8 zeigt eine Ansicht von vorne, wobei der Fräser 1 vertikal steht. Dabei ist auch nochmals die Knochenklammer 26 erkennbar, wobei der mittlere Klammeransatz 28 über eine Führung 35 in Richtung des Doppelpfeils 36 zum Festlegen bzw. Lösen des Knochens verstellbar ist.

In Fig. 8 ist eine auf einen selbsthemmenden Schneckentrieb einwirkende Einstellschraube 37 zur Einstellung der Inklination in Verbindung mit einer feststehenden Markierung 38 und einer schwenkbaren Skala 39 dargestellt.

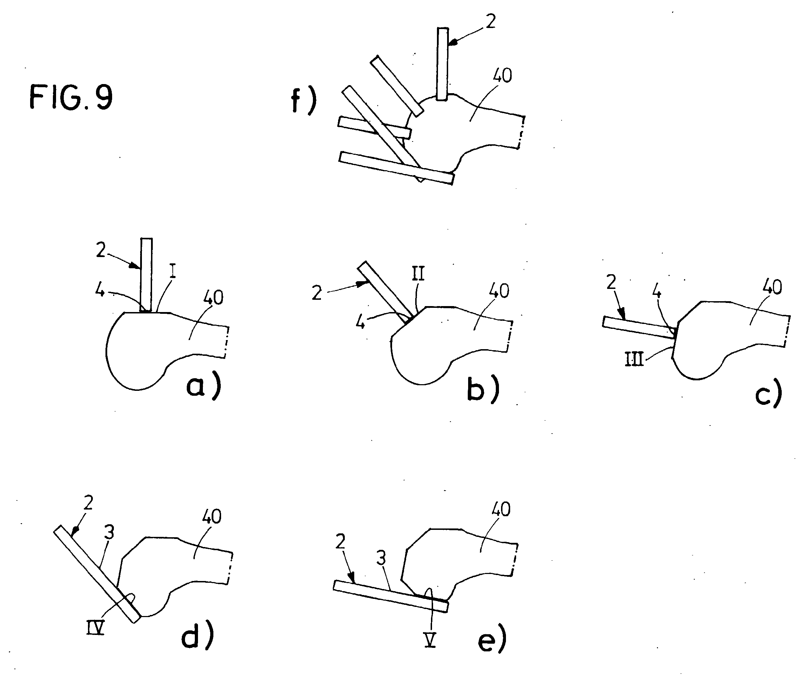

In Verbindung mit Fig. 9 wird nachfolgend die Arbeitsweise des erfindungsgemäßen Instrumentariums nochmals näher erläutert:

Wie in Fig. 9a dargestellt, wird die erste Schnittebene I im Wege des Stirnfräsens erstellt, wobei die Stirnfläche 4 des Fräskopfes 2 senkrecht auf der Schnittebene I steht. Das Instrumentarium befindet sich dabei in der in Fig. 2 dargestellten Einstellung.

Als nächstes wird, wie in Fig. 9b veranschaulicht, ebenfalls im Wege des Stirnfräsens, die Schnittebene II erstellt, wobei sich das Instrumentarium in der Einstellung gemäß Fig. 4 befindet.

Anschließend wird im Wege des Stirnfräsens die Schnittebene III erstellt. Das Instrumentarium befindet sich in der Einstellung gemäß Fig. 1.

Daran anschließend erfolgt eine Umstellung von Stirnfräsen auf Walzenfräsen, wie dies in Fig. 6 gegenüber Fig. 5 veranschaulicht ist. Es wird nun die Mantelfläche 3 des Fräskopfes 2 zum Fräsen eingesetzt und diese längs der Schnittebene IV, d.h. parallel zu dieser verfahren, wie dies in Fig. 9d veranschaulicht ist. Anschließend erfolgt im Wege des Walzenfräsens, wie in Fig. 9e veranschaulicht, die Erstellung der Schnittebene V.

In Fig. 9f ist in der Zusammenstellung nochmals dargestellt, wie die einzelnen Schnittflächen I bis V der Reihe nach erstellt werden.

## Patentansprüche

1. Instrumentarium zur Herstellung der Befestigungsflächen zur Befestigung einer Kniegelenkendoprothese an dem knienahen Oberschenkel und am Tibiakopf, **gekennzeichnet durch** einen rotierenden Fräskopf (2) der relativ zur Längsachse von Femur (40) bzw. Tibia mittels Befestigungseinrichtungen (Knochenklammer 26) festlegbar und relativ zu dieser Längsachse unter Definition von Fräsebenen (Schnittebenen I bis V) durch Verschwenken endlagenstabil winkeleinstellbar ist und in der jeweils so eingestellten Fräsebene (Schnittebenen I bis V) diese abdeckend als Stirn- bzw. Walzenfräser arbeitend verfahrbar gelagert ist.

2. Instrumentarium nach Anspruch 1, **dadurch gekennzeichnet, daß** die Befestigungseinrichtungen den jeweiligen Femur (40) umgreifende Klammern (27, 28, 29) umfassen.

3. Instrumentarium nach Anspruch 2, **dadurch gekennzeichnet, daß** eine erste Klammer den Femur (40) von oben übergreift, wobei die Klammer (26) Klammeransätze (27, 28, 29) aufweist, die relativ zueinander verschiebbar und endlagenfixierbar sind.

4. Instrumentarium nach Anspruch 3, **dadurch gekennzeichnet, daß** an den Enden der Klammeransätze (27, 28, 29), Befestigungsdorne (30) und/oder Bohrungen zum Einbringen von Befestigungsschrauben ausgebildet sind.

5. Instrumentarium nach Anspruch 1, **dadurch gekennzeichnet, daß** der Fräskopf (2) höhenverstellbar ausgebildet ist.

6. Instrumentarium nach Anspruch 5, **dadurch gekennzeichnet, daß** zur Höhenverstellung eine Einstellschraube (31) für eine endlagenstabile Gewindespindel vorgesehen ist.

7. Instrumentarium nach Anspruch 1, **dadurch gekennzeichnet, daß** der Fräskopf (2) in den Fräsebenen über Schienenführungen (5, 11) linear translatorisch verlagerbar und über eine Schwenkachse (16) oder Kreuzsupport verschwenkbar ist.

8. Instrumentarium nach Anspruch 1, **dadurch gekennzeichnet, daß** eine Voreinstelleinrichtung (17, 18) für die Beinachsenverstellung vorgesehen ist.

9. Instrumentarium nach Anspruch 1, **dadurch gekennzeichnet, daß** eine Beinlängeneinstelleinrichtung (Stellschraube 31) vorgesehen ist.

10. Instrumentarium nach Anspruch 1, **dadurch gekennzeichnet, daß** eine Prothesengrößeneinstelleinrichtung vorgesehen ist.

11. Instrumentarium nach einem der Ansprüche 1 bis 10, **dadurch gekennzeichnet, daß** die Einstelleinrichtungen und/oder die Bewegungen des Fräskopfes (2) in den Fräsebenen (I bis V) servomotorisch angetrieben sind.

12. Instrumentarium nach Anspruch 11, **dadurch gekennzeichnet, daß** die Ansteuerung der Servomotoren computergesteuert erfolgt.

13. Instrumentarium nach Anspruch 12, **dadurch gekennzeichnet, daß** zur Navigation Infrarotmarker vorgesehen sind.
